Europäisches Patentamt

(19) European Patent Office

Office européen des brevets

(11) Veröffentlichungsnummer: **0 081 683 B1**

# (12) EUROPÄISCHE PATENTSCHRIFT

(45) Veröffentlichungstag der Patentschrift:
05.02.86

(51) Int. Cl.⁴: **C 07 C 1/20**, **C 07 C 11/02**, **C 07 C 4/04**

(21) Anmeldenummer: 82110415.5

(22) Anmeldetag: 11.11.82

(54) Verfahren zur Herstellung von Olefinen durch Umsetzung von Methanol und/oder Dimethylether.

(30) Priorität: 19.11.81 DE 3145712

(43) Veröffentlichungstag der Anmeldung:
22.06.83 Patentblatt 83/25

(45) Bekanntmachung des Hinweises auf die Patenterteilung:
05.02.86 Patentblatt 86/6

(84) Benannte Vertragsstaaten:
BE DE FR GB NL

(56) Entgegenhaltungen:
EP - A - 0 016 406
EP - A - 0 017 027
EP - A - 0 054 375
EP - A - 0 074 075
GB - A - 1 481 766
US - A - 4 197 418

(73) Patentinhaber: BASF Aktiengesellschaft,
Carl-Bosch-Strasse 38, D-6700 Ludwigshafen (DE)

(72) Erfinder: Marosi, Laszlo, Dr., Leuschnerstrasse 32,
D-6700 Ludwigshafen (DE)
Erfinder: Stabenow, Joachim, Dr., Am Feldrain 22,
D-6940 Weinheim (DE)
Erfinder: Schilmper, Hans-Ulrich, Dr., Im Erlich 74,
D-6720 Speyer (DE)
Erfinder: Schwarzmann, Matthias, Dr.,
Carl-Bosch-Strasse 54, D-6703 Limburgerhof (DE)
Erfinder: Ostertag, Werner, Dr., Oberer-Bergel-Weg 2,
D-6718 Gruenstadt (DE)

## Beschreibung

Die Erfindung betrifft ein Verfahren zur Herstellung von $C_2$-$C_4$-Olefinen aus Methanol und/oder Dimethylether.

In neuerer Zeit gewinnen Bemühungen, Methanol zur Herstellung von Olefinen zu verwenden, zunehmendes Interesse. Methanol läßt sich aus Kohle, über Kohlevergasung und Herstellung von Sythesegas, mit Hilfe bewährter Technologien leicht herstellen. Gelingt es, Methanol in wirtschaftlicher Weise in niedere Olefine umzuwandeln, können die heute üblichen Weiterverarbeitungsverfahren der chemischen Industrie auch bei der Verwendung von Kohle als Rohstoff weiter beibehalten werden. In den vergangenen Jahren sind daher mehrere Verfahren entwickelt worden, die die Umwandlung von Methanol und/oder Dimethylether in Olefine zum Gegenstand haben. Die bisher bekanntgewordenen Verfahren haben den Nachteil, daß sie nur geringe Umsätze an Methanol ergeben, oder ihre technische Durchführbarkeit schwierig ist. Unter technisch realisierbaren Reaktionsbedingungen ergaben Zeolithkatalysatoren . vom Pentasil-Typ überwiegend flüssige Kohlenwasserstoffe mit einem hohen Aromatenanteil. Andere Zeolithsorten, z. B. Chabazit, ergeben zwar hohe Olefinausbeuten, haben aber eine zu geringe Lebensdauer.

Es wurde nun gefunden, daß man diese Schwierigkeiten bei der Herstellung von $C_2$-$C_4$-Olefinen in zwei Stufen durch Umsetzung von Methanol und/oder Dimethylether am Zeolithkatalysatoren vom Pentasiltyp bei einer Temperatur zwischen 300 und 600°C und anschließendes Cracken eines Teils der Umsetzungsprodukte vermeidet, wenn man in der ersten Stufe das Rohmethanol und/oder den Dimethylether an einem Borosilikatzeolith oder einem Aluminosilikatzeolith, der mindestens 4 Stunden bei Temperaturen über 400°C mit Wasserdampf behandelt wurde, mit einer Katalysatorbelastung von 0,1 bis 100 g Methanol und/oder Dimethylether/g Kat. h und einem Druck von 1 bis 3 bar zu mindestens 95 % in eine Mischung von Kohlenwasserstoffen umsetzt, die weniger als 15 Gew.-% aromatische Verbindungen enthält, die gebildeten $C_2$-$C_4$-Olefine abtrennt und die $C_5$+Kohlenwasserstoffe zusammen mit den $C_2$-$C_4$-Paraffinen in Gegenwart von Wasserdampf thermisch spaltet.

Aufgabe der Erfindung war es insbesondere, bei der Umsetzung in erster Stufe, d. h. vor der Wasserdampfspaltung, ein Gemisch von Kohlenwasserstoffen zu erzeugen, das möglichst wenig aromatische Anteile enthält. Der Anteil der Aromaten soll unter 15 %, vorteilhaft 0 bis 12 % betragen.

Im allgemeinen arbeitet man bei Katalysatorbelastungen von 0,1 bis 100 g, vorzugsweise bei 1 bis 12 Methanol und/oder Dimethylether/g. Kat h, Temperaturen von 300 bis 800 °C insbesondere 350 bis 500 °C, bei normalem oder erhöhtem Druck, z. B. bei 1 bis 3 bar.

Zweckmäßigerweise wird die Umsetzung in der ersten Stufe in einem Wirbelschichtreaktor ausgeführt. Dabei dient als Wirbelmittel der Strom der gasförmigen Ausgangsstoffe; man kann aber auch zusätzlich Wirbelmittel, z. B. Stickstoff oder Wasserdampf, einführen.

Nach der ersten Stufe werden aus dem erhaltenen Kohlenwasserstoffgemisch die $C_2$ bis $C_4$-Olefine, z. B. durch Destillation, abgetrennt, während der übrige Teil, die $C_5$+-Kohlenwasserstoffe und $C_2$-$C_4$-Paraffine mit dem geringen Aromatenanteil der Wasserdampfspaltung in herkömmlicher Weise unterworfen werden.

## Beispiel

Es werden drei Zeolithe vom Pentasil-Typ hergestellt: zwei Borosilikatzeolithe mit $SiO_2$/$B_2O_3$-Molverhältnissen von 33 bzw. 145 und ein Aluminosilikatzeolith mit einem $SiO_2$/$Al_2O_3$ Molverhältnis von 30. Die Aminkomponente wird durch Abbrennen bei 530 °C aus den Zeolithen entfernt. Der Aluminosilikatzeolith wird anschließend bei 430 °C 70 Stunden mit Wasserdampf behandelt.

Die Zeolithe wurden dann in einem Gewichtsverhältnis Zeolith: $Al_2O_3$ = 60 : 40 mit Böhmit-verstrangt und ein Teil der erhaltenen Stränge zu einem Wirbelbettkatalysator mit einer Körnung von 0,1 - 0,4 mm verarbeitet. Man erhält drei Katalysatoren:

Katalysator 1: Borosilikatzeolith,          $SiO_2$/$B_2O_3$ = 31
Katalysator 2:          "          ,          $SiO_2$/$B_2O_3$ = 145
Katalysator 3: Aluminosilikatzeolith,          $SiO_2$/$Al_2O_3$ = 30
          dampfbehandelt.

Die hergestellten Katalysatoren werden auf ihre katalytische Aktivität bei der Umwandlung von Methanol in Olefine getestet. Testbedingungen und die Zusammensetzung der erhaltenen Kohlenwasserstoffe sind in der folgenden Tabelle zusammengestellt:

## Tabelle

| Versuch | a) | b) | c) | d) | e) |
|---|---|---|---|---|---|
| Katalysator | 2 | 3 | 1 | 1 | 1 |
| Reaktionstemperatur (°C) | 370 | 500 | 500-520 | 500-520 | 400-420 |
| Belastung (g $CH_3OH$/g Kat., h) | 3,2 | 4 | 3 | 1 | 0,5 |
| Raumgeschwindigkeit (cm/sec) | — | — | 13,3 | 4,4 | 2,0 |
| Methanolkonzentration (%) | 83 | 83 | 80 | 80 | 80 |
| Reaktortyp | adiaba-tisch | adiaba-tisch | Wirbel-reaktor | Wirbel-reaktor | WB |
| Druck | 1,3 | 1,3 | 1,5 | 1,5 | 1,5 |
| Umsatz zu Kohlenwasserstoffen (%) | 96 | 100 | 80 | 96 | 100 |
| C-Gehalt am Katalysator (%) | — | — | 15-18 | 15-18 | 9-10 |
| **Reaktionsprodukte (Gew.-%)** | | | | | |
| Ethylen | 6,45 | 6 | 5,5 | 7 | 4,5 |
| Propylen | 43,5 | 35 | 20 | 24 | 19 |
| Butene | 13,1 | 24 | — | — | — |
| Ethen | — | <1 | 0,6 | 1,0 | 0,2 |
| Propan | 2,81 | <3 | 2,6 | 4 | 3,3 |
| Butan | 6,85 | 6 | — | — | — |
| $C_5^+$-KW-e | 25 | 25 | 30-31 | 38-40 | 42-43 |
| Aromaten | 10 | 10 | — | — | — |
| $\Sigma\ C_4$ | — | — | 10-11 | 19 | 15-16 |

Darauf werden aus dem Produktstrom des Versuches 2 d) die $C_2$-$C_4$-Olefine und Methan abgetrennt und die gesättigten $C_2$-$C_C$-Kohlenwasserstoffe zusammen mit den $C_5^+$-Kohlenwasserstoffen bei 820°C dem 'steam-crack'-Prozeß unterworfen. Der Gesamtethylenwert steigt durch diese Behandlung von 7 auf 14,2 Gew.%, der Propylenwert von 24 auf 26,2 Gew.%. Der Anteil der $C_5^+$-Kohlenwasserstoffe beträgt 23 Gew.%.

## Patentansprüche

1. Verfahren zur Herstellung von $C_2$-$C_4$-Olefinen in zwei Stufen durch Umsetzung von Methanol und/oder Dimethylether an Zeolithkatalysatoren vom Pentasiltyp bei Temperaturen zwischen 300 und 600°C, und anschließendes Cracken eines Teils der Umsetzungprodukte, dadurch gekennzeichnet, daß man in der ersten Stufe das Rohmethanol und/oder den Dimethylether an einem Borosilikatzeolith oder einem Aluminosilikatzeolith, der mindestens 4 Stunden bei Temperaturen über 400°C mit Wasserdampf behandelt wurde, mit einer Katalysatorbelastung von 0,1 bis 100 g Methanol und/oder Dimethylether/g Kat . h und einem Druck von 1 bis 3 bar zu mindestens 95 % in eine Mischung von Kohlenwasserstoffen umsetzt, die weniger als 15 Gew.% aromatische Verbindungen enthält, die gebildeten $C_2$-$C$-Olefine abtrennt und die $C_5^+$-Kohlenwasserstoffe zusammen mit den $C_2$-$C_4$-Paraffinen in Gegenwart von Wasserdampf thermisch spaltet.

2. Verfahren nach Anspruch 1, dadurch gekennzeichnet, daß die Umsetzung von Methanol und/oder Dimethylether in der ersten Stufe in der Wirbeschicht ausgeführt wird.

## Claims

1. A process for the preparation of $C_2$-$C_4$-olefins in two stages by converting methanol and/or dimethyl ether over a zeolite catalyst of the pentasil type at from 300 to 600°C and then subjecting some of the products to a cracking procedure, wherein, in the first stage, at least 95% of the crude methanol and/or the dimethyl ether is converted over a borosilicate zeolite or an aluminosilicate zeolite which has been treated with steam for at least 4 hours at above 400°C, using a catalyst load of from 0.1 to 100 g of methanol and/or dimethyl ether per g of catalyst per hour, and a pressure of from 1 to 3 bars, into a mixture of hydrocarbons which contains less than 15% by weight of aromatic compounds, and the $C_2$-$C_4$-olefins formed are isolated and the $C_5^+$ hydrocarbons together with the $C_2$-$C_4$-paraffins are subjected to thermal cleavage in the presence of steam.

2. A process as claimed in claim 1, wherein the conversion of methanol and/or dimethyl ether in the first stage is carried out in a fluidized bed.

## Revendications

1. Procédé de préparation d'oléfines en $C_2$ à $C_4$ par une réaction en deux stades de l'alcool méthylique et(ou) de l'éther diméthylique à des températures comprises entre 300 et 600°C en présence de catalyseurs zéolithiques du type pentasil, suivie du craquage d'une partie des produits réactionnels, caractérisé en ce que l'on transforme, dans le premier stade, le méthanol brut et(ou) l'éther diméthylique sur une zéolithe boro-silicatée ou une zéolithe alumino-silicatée, traitée pendant au moins quatre heures à des températures supérieures à 400°C avec de la vapeur d'eau, le catalyseur étant charge de 0,1 à 100 g de méthanol et(ou) d'éther diméthylique par g de catalyseur et par heure, sous une pression de 1 à 3 bars, jusqu'à au moins 95 % en un mélange d'hydrocarbures, qui contient moins de 15 % en poids de composés aromatiques, on separe ensuite les oléfines en $C_2$ à $C_4$ formées, puis on soumet les hydrocarbures en $C_5$ et plus avec les paraffines en $C_2$ à $C_4$ à un craquage thermique en présence de vapeur d' eau.

2. procédé suivant la revendication 1, caractérisé en ce que le réaction du méthanol et(ou) de l'éther diméthylique dans le premier stade est réalisée dans un lit fluidisé.